# EUROPEAN PATENT APPLICATION

(11) **EP 1 462 800 A1**
(43) Date of publication of application: **29.09.2004**
(21) Application number: 02804657.1
(22) Date of filing: 09.12.2002
(51) Int. Cl.: G01N 33/48, G01N 33/49

(54) **BLOOD CELL SEPARATION SYSTEM**

(30) Priority: 11.12.2001 JP 2001377055
(71) Applicant: Netech Inc., Kawasaki-shi, Kanagawa 213-0012 (JP)
(72) Inventor: YURA, Hirofumi, Kawasaki-shi, Kanagawa 213-0011 (JP); SAITO, Yoshio, Yokohama-shi, Kanagawa 236-0011 (JP); KITAGAWA, Michihiro, Tokyo 158-0082 (JP)
(74) Representative: Poulin, Gérard
(86) International application number: PCT/JP2002/012839
(87) International publication number: WO 2003/050532

(57) **Abstract**

A blood cell separating system is offered for precisely separating and concentrating rare fetal nucleated cells intermixed in the blood of a pregnant woman, to conveniently obtain test preparations capable of being used for prenatal chromosomal/genetic diagnosis. A blood cell separating system is characterized by comprising (1) a primary separating device for removing mainly non-nucleated erythrocytes, leukocytes and platelets from blood samples taken from a pregnant woman to obtain a primary separated sample, (2) a secondary separating device for using a carbohydrate-lectin method to remove residual non-nucleated erythrocytes and leukocytes from the primary separated sample obtained by the primary separating device to obtain a secondary separated sample with concentrated fetal nucleated cells, and (3) preparing device for preparing the secondary separated sample obtained by the secondary separating device.

## Description

### Technical Field

The present invention relates to a system for the separation of blood cells using lectins, particularly to a system for efficiently removing non-nucleated erythrocytes and mature leukocytes, to separate and concentrate fetal nucleated erythroblasts, from samples containing nucleated erythroblasts which are fetal nucleated cells found in the maternal peripheral blood or umbilical blood of pregnant women, and further, a system for testing the chromosomes and genes thereof.

### Background Art

In the field of genetic diagnosis, the development of methods for prenatal diagnosis which do not endanger the embryo, *i*.*e*. the fetus, has been long anticipated. The genetic diagnosis methods which are practiced clinically at present are invasive procedures such as amniocentesis, villus sampling and fetal blood collection, of which sampling of fetal cells by amniocentesis in particular allows for a positive diagnosis, but also carries a high risk of miscarriage at about 1/300.

Additionally, while methods for predicting fetal abnormalities from changes in maternal serum biochemical markers present in the maternal peripheral blood such as AFP (α-fetoprotein), hCG (chorionic gonadotropin), uE3 (uric Estriol 3, a type of estrogen secreted from the fetal adrenal glands during pregnancy and used in tests of fetal-placental function) and inhibin A (a type of gonadotropin) are desirable for being non-invasive, they are only methods for screening for the need to perform amniocentesis and cannot be relied upon to make any positive diagnoses.

On the other hand, clinical studies on the incompatibility of blood types between mother and child have made it dear that fetal cells can find their way into the maternal circulatory system. Thus, if intermixed fetal cells can be isolated from the maternal blood, and if they are nucleated cells, their DNA and chromosomes can be used to safely make positive fetal diagnoses. In 1969, Walknowska *et al*. reported that they had cultured maternal peripheral lymphocytes and discovered a 46XY karyotype in 21 of 30 women pregnant with male children. However, the work of extracting lymphocytes having a Y chromosome from an entire group of similar lymphocytes is extremely difficult, and attempts to separate and concentrate them by means of a nylon wool column or density centrifugation did not succeed in yielding a practical solution. Additionally, the presence of fetal cells remaining from past pregnancies was also considered to be a problem, and the development of a safe method of positive fetal diagnosis allowing for testing of a desired neonatal fetus did not progress any further.

In recent years, nucleated erythroblasts which are cells that have a short lifespan and are normally almost non-existent in the mother have garnered some attention as fetal nucleated cells present in the maternal blood, and research into the possibility of their use as cells to be used for diagnosis has become very active. However, the proportion of their presence in the maternal blood is extremely low at 1/10⁵ to 1/10⁷ of all nucleated cells, and just as with fetal cell diagnosis by lymphocytes, there are technical obstacles to achieving a process of separating and detecting a sufficient number of fetal nucleated erythroblasts from maternal nucleated cells such as leukocytes.

While attempts have been made to separate the erythroblasts by means of flow cytometry using transferin receptor antibodies or magnetic beads, the problem of specificity of antibodies has precluded the efficient detection of erythroblasts having Y chromosomes.

On the other hand, there are collection methods in which, instead of performing cell separation, the erythroblasts are identified morphologically and picked out one at a time by micromanipulation, but the process of discriminating erythroblasts from among masses of maternal nucleated cells requires considerable technical expertise and is extremely time-consuming. Thus, this method requires special equipment, and for example, can take several days to process a single specimen. At present, perhaps a single erythroblast will be detected from 1 cc of maternal peripheral blood. However, at least 30 fetal cells are considered to be necessary in order to establish a general and practical fetal testing method for testing genes and chromosomes in a statistically conclusive manner, which makes this sampling method unrealistic when bearing in mind that the quantity of blood which may be safely collected from a pregnant woman is at most 10 cc, so that even to this day, there is a strong demand for a method for quickly and conveniently separating and concentrating erythroblasts at a high yield.

The present inventors have been performing research with a focus on the specific interactions of carbohydrate chains, and have obtained a patent (Japanese Patent No. 3139957) on a cell culture matrix having a matrix surface of a dish or the like coated with glycoconjugate polymers having various carbohydrates as side chains, whereby lectins which have an affinity to these carbohydrate chains are selectively attached. Furthermore, they discovered that a separating method using lectins can be used to separate and concentrate not only erythroblasts, but also immature hematopoietic cells, thus making it possible to detect 10-30 fetal nucleated cells (International Publication WO00/58443).

When separating and recovering blood cells such as erythroblasts, the peripheral blood is generally first pretreated using density centrifugation, but the erythroblasts were found to have been damaged during this pretreatment stage by density centrifugation. For example, the specific gravity of nucleated erythroblasts changes between about 1.077-1.095, and when a high-density fluid (such as Ficoll or the like) with a certain specific gravity is used, erythroblasts are sometimes detected from both the erythrocyte layer under the Ficoll layer and above the Ficoll layer, so that simple separation by density centrifugation can cause some of the erythroblasts to be lost. This is thought to be due to the uncertainty in the specific gravity of fetal erythroblasts which contain fetal hemoglobin and are about to denucleate.

Furthermore, in order to know of chromosome abnormalities and the like in nucleated erythroblasts separated and purified in this way, they must be inspected by the FISH (Fluorescence In Situ Hybridization) method or the like, but this gives rise to practical problems which need to be overcome during the process of preparing specimens appropriate for such testing.

Therefore, in order to completely resolve the above-described problems, the present invention has the object of providing a new blood cell separating system capable of being offered for clinical practice, based on precision separation and recovery of nucleated erythrocytes (NRBC) using lectins. That is, the present invention offers a system which considerably prevents the loss of erythroblasts during the pretreatment step while using lectins to precisely separate the rare fetal nucleated cells from the maternal cells in a maternal blood sample of a limited quantity, thereby to separate, concentrate and recover the desired immature fetal cells or NRBC's selectively and at a high yield, and to offer a method of producing a test preparation containing fetal cells separated and recovered using such a system.

### Summary of the Invention

The blood separating system of the present invention
(1) comprises a primary (crude) separating device for removing mainly non-nucleated erythrocytes, leukocytes and platelets from a blood sample taken from a pregnant woman to obtain a primary (crude) separated sample, said primary separating device separating and removing mainly leukocytes which are nucleated cells, non-nucleated erythrocytes, platelets and the like under conditions which highly prevent loss of fetal nucleated cells among the various types of blood cells included in a maternal blood sample;
(2) comprises a secondary (precision) separating device for removing residual non-nucleated erythrocytes and leukocytes from a primary separated sample obtained from said primary separating device, to obtain thereby a secondary (precision) separated sample with concentrated fetal nucleated cells, the secondary separating device involving incubation of said primary separated sample, under conditions which inactivate the cells, along with a predetermined concentration of lectins, on a substrate having glycoconjugate polymers affixed to the surface thereof, thereby selectively binding fetal nucleated cells contained in said primary separated sample with said lectins to thereby concentrate and attach them to said substrate by means of a lectin-carbohydrate interaction to form a secondary separated sample; and
(3) comprises a preparing device for preparing a secondary separated sample obtained from said secondary separating device, the preparing device involving centrifugation, under predetermined conditions, of said substrate on which said fetal nucleated cells have been concentrated and attached, to thereby obtain a test preparation wherein fetal nucleated cells from maternal blood are present in a state advantageous to genetic/chromosomal testing by the FISH method or the like, at levels which allow for a positive diagnosis.

The blood separating system of the present invention should preferably further comprise a testing device for using a test preparation obtained from the aforementioned preparing device to test the chromosomes and/or genes in fetal nucleated cells contained in the test preparation.

Furthermore, the present invention also offers a method of producing a test preparation for a prenatal fetal diagnosis using the above-described blood cell separating system, as well as a test preparation produced by this production method, wherein immature fetal cells are present in a state advantageous to genetic/chromosomal testing by the FISH method or the like, at levels which allow for a positive diagnosis.

### Brief Description of the Drawings

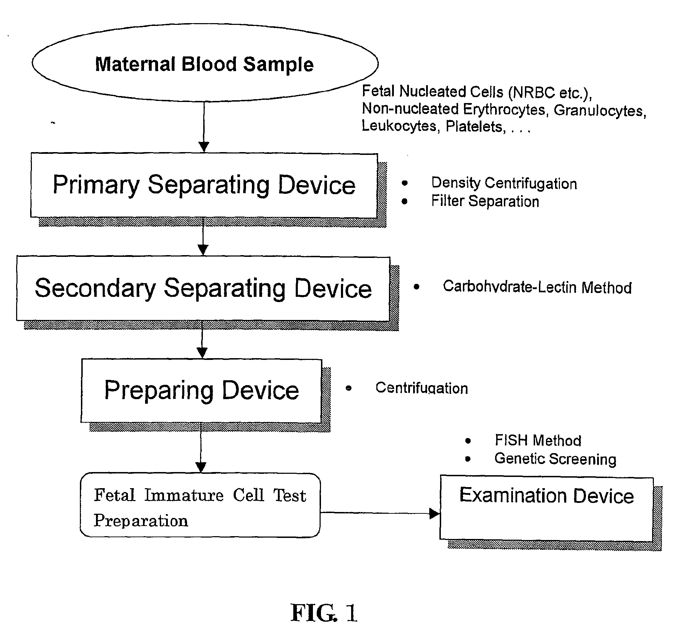

Fig. 1 is a block diagram showing an example of a blood cell separating system of the present invention.
Fig. 2 is a microscopic (X200) photograph showing blood cells immobilized on a glycoconjugate-polymer-coated substrate from maternal blood using 300 µg/ml of lectin (SBA).
Fig. 3 is a microscopic (X200) photograph showing blood cells immobilized on a glycoconjugate-polymer-coated substrate from maternal blood using 8 µg/ml of lectin (SBA).
Fig. 4 is a microscopic (X1000) photograph showing the immobilized cells shown in Fig. 3.
Fig. 5 is a cross-section diagram showing an example of a substrate usable in blood cell separating system of the present invention.

### Descriptions of Preferred Embodiments

Herebelow, the present invention shall be described in detail.

First, a blood sample to be used in the system and method of the present invention is taken from a pregnant woman. As blood collecting means, a blood collecting tool which is generally used, such as a syringe, vacuum blood collecting tube or a blood bag may be used. The blood to be taken may be any type of blood including venous blood, umbilical blood, intervillous blood from the placenta or myeloid fluid, but in order to limit the intrusion into the maternal body as much as possible, peripheral venous blood is the most preferable. While there is no particular restriction on the quantity of the blood sample, it should generally be about 1-10 ml in view of the problems posed by invasion of the maternal body. In order to prevent the taken blood sample from coagulating, EDTA, heparin, CPD/ACC or the like should preferably be added. In order to obtain meaningful results, it would be preferable to use a fresh blood sample taken from the pregnant woman within 24 hours, more preferably within 6 hours before use.

The blood cell separating system of the present invention comprises a primary separating device for mainly removing a large portion of the non-nucleated erythrocytes and maternal leukocytes which are a cellular fraction to be removed from the above-referenced blood sample. Specifically, the primary separating device should preferably be a density centrifugation device using a liquid having a specific density or a filter separation device.

The first embodiment of a primary separating device forming the blood cell separating system of the present invention is a density centrifugation device, wherein a density gradient fluid set to a predetermined density using an existing density adjusting agent such as sucrose and modified sucrose, glycerol or colloidal silica coated with polyvinylpyrrolidone is used. Examples include Ficoll-Paque, Ficoll-Hypaque and Percoll available from Pharmacia, or Histopaque available from Asuka-Sigma. The density gradient fluids generally used in the past for separation of blood components from the whole blood are set to a specific gravity of 1.077 (such as Ficoll-Paque and Histopaque-1077). In this case, centrifugation causes granulocytes and erythrocytes with a high specific gravity to go underneath the density gradient fluid, while monocytes and lymphocytes such as leukocytes rise above the density gradient fluid. However, the present inventors discovered that when using the density gradient fluid with a specific gravity of 1.077, a portion of the fetal nucleated cells such as NRBC can spread into the density gradient fluid after centrifugation, so that much of the rare fetal nucleated cells are lost if only the layer containing the monocytes is taken, as is conventional. Thus, in the separating system of the present invention, a density gradient fluid having a specific gravity higher than 1.077 is preferably used as the primary separating device, and this can be used to prevent loss of fetal nucleated cells.

As shown by the below-described examples, increasing the specific gravity of the density gradient fluid will increase the recovery rate of fetal nucleated cells, but will simultaneously result in an increase in the intermixture of unwanted leukocytes. However, since the present invention can have a panning device as well as a secondary separating device which makes use of carbohydrate-lectin interactions as shall be described below, the intermixed leukocytes can be separated and removed to recover more fetal nucleated cells. Therefore, the density centrifugation device of the present invention comprises a centrifugal tube, a density gradient fluid with a specific gravity of 1.077-1.105, preferably 1.080-1.095 and more preferably 1.090-1.095 g/cm³ which is placed in the centrifugation tube, and a centrifuge separator. A blood sample taken from a pregnant woman is put into a centrifugation tube in which the density gradient fluid has been placed, and this is placed in the centrifuge separator to undergo centrifugation for 20-40 minutes at 500-2000 rpm (50-800 G), whereby a primary separated sample is obtained as a fraction of a layer placed above the density gradient fluid which contains large amounts of fetal nucleated cells.

In some cases, the primary separating device of the present invention may also have a panning device for further removing the intermixed leukocytes and the like. This panning device comprises, for example, aplate or the like such as a polystyrene dish which has been surface-treated with fetal calf serum (FCS), for separating and removing leukocyte components such as monocytes and granulocytes by making use of non-specific adhesion with respect to the plate.

The specific panning process may be such as has been conventionally used in this field. For example, the sample can be provided on the dish and incubated for a predetermined period, then the cells which have not adhered to the plate can be recovered as a suspension.

The dish should preferably be of a disposal plastic type, of which any type including commercially available plastic plate products such as the polystyrene dishes available from Nunc, Falcon, Iwaki Seiyaku or Sumitomo Bakelite may be used. FCS is added to these plates, then let stand, for example, for 2 hours at 4 °C, to coat the surface with proteins in the FCS. As a result, the hydrophobic plastic surface becomes hydrophilic, but any conditions can be used as long as a temperature of more than 37 °C at which the protein components in the FCS begin to change, or a temperature which is below freezing is not used. Aside therefrom, it is also effective to use carbohydrates as the coating agents, of which PV-sugars (product of Netech, sold by Seikagaku Kogyo) which are easily coated onto plastic surfaces are suitable for use. The coating is obtained by dissolving PV-sugar powder into distilled water to obtain a 10-1000 µg/ml solution, of which those of 50-500 µg/ml are preferably used. This PV-sugar solution is put into the dish and left at room temperature for at least 30 minutes, whereupon the PV-sugar is adsorbed and the dish surface is hydrophilized. Examples of carbohydrates which can form a PV-sugar include, as glucose types, PV-G (glucose), PV-MA (maltose), PV-GA (gluconic acid), PV-CA (cellobiose) and PV-Lam (laminaribiose); as galactose types, PV-LA (lactose), PV-LACOOH (carboxylated lactose) and PV-MeA (melibiose); as well as PV-Man (mannobiose) and PV-GlcNAc (N-acetylchitobiose), but they may be of any type including naturally occurring polysaccharides, as long as they are carbohydrates which are capable of efficiently coating the dish surface. These are preferably used because they can easily coat polystyrene dishes, and do not lose or damage many fetal cells. In particular, those in which the carbohydrates forming the glycoconjugate polymer material are gluconic acid (PV-GA), melibiose (PV-MeA) or mannose (PV-Man) are particularly advantageous for their ability to achieve a high leukocyte adsorption and removal process in which the loss of fetal cells is held low. As coating agents, PV-sugars are preferable also for being synthetic polymers having a separating performance which is equivalent or superior to that of FCS, and being easier to obtain in stable lots and more easily stored than FCS.

After centrifugation, the primary separated sample is placed in the hydrophilized dish, and by letting stand for at least 5 minutes, the leukocyte components can be made to adhere at a higher rate to the dish surface, but they should preferably be left for 15 minutes to 2 hours in order to achieve an appropriate rate of leukocyte adherence and removal. During this period, any temperature at which the cells contained in the primary separated sample do not die may be used, but a temperature of 18-37 °C at which the leukocytes can be actively led to adhesion is preferably used.

The panning process is not restricted to use of a plastic plate, and a glass plate or the like can be used by coating with FCS or PV-sugars as described above.

In general, contamination of a blood sample with many platelets, which can bind to lectins used in a secondary separation, after centrifugation may occur, which would adversely affect of the preciseness of erythroblast analysis. However, panning treatment can effectively remove by adhering the platelets contaminated during blood sample preparation. Therefore, panning is a process effective for pre-removing excess granulocytes, monocytes and platelets, and offers highly precise erythrocyte separation.

Due to these panning treatments, even platelets, which have an extremely small specific gravity and grain size but an extremely high adhesion, are automatically removed from the primary separated sample obtained by the above-described centrifugation device so as to decrease to less than the detectable limit, thus making the sample rich in fetal nucleated cells with only a few non-nucleated erythrocytes and leukocytes such as lymphocytes remaining, and this can be made into the primary separated sample for the separating system of the present invention.

A second embodiment of a primary separating device forming the blood cell separating system of the present invention is a filter separating device. This filter separating device has a porous filter having a predetermined pore diameter generally used for separation of blood components, such that by passing a blood sample through this filter, the non-nucleated erythrocyte components which are deformable are normally passed through and removed, whereas the components including leukocyte components and erythroblasts, which are fetal nucleated cells, remain inside or on top of the filter material. By recovering the components remaining in the filter by means of a detergent fluid or the like, a primary separated sample containing fetal nucleated cells and leukocytes can be obtained.

The filter used herein is not especially restricted as long as it is capable of passing non-nucleated erythrocyte components (average size 7-8.5 µm), but physically or chemically capturing to block passage of leukocytes (average size 7-30 µm) and fetal nucleated cells (average grain size 8-19 µm), such that anything can be used, for example, non-woven fabrics which serve to capture by the form of aggregation or size of the fibers forming the filter, porous films with pore diameters controlled by elongating polymers, beads or spongy materials, as long as they have pore sizes within a range such as to capture orthochromatic erythroblasts having a minimum size of about 8 µm which are the most likely in the group of nucleated erythroblasts to be the object of recovery, yet allow non-nucleated erythrocytes to pass through by deformation. The material of the filter is not especially restricted, and may be of a synthetic or natural polymer such as fluoropolymers, polysulfones, polyesters, polyvinylacetals, polyvinylalcohols, polyamides, polyimides, polyurethanes, polyacrylics, polystyrenes, polyketones, silicones, polylactates, celluloses, chitosans, celluloses, silk or hemp, or inorganic materials including hydroxyapatite, glass, alumina, titania or metals such as stainless steel or titanium aluminum, which retain a certain level of adhesion with respect to leukocytes for maintaining the ability to collect leukocytes. Polyamides such as nylon, polyester, polyurethane, polyethylene, polypropylene, acrylic, polystyrene, polycarbonate, cellulose and hydroxyapatite are preferable in view of cost and production. The pore size of the filter should be 1.0-40 µm, preferably 2.0-20 µm and more preferably 3.0-10 µm, and if the filter is composed of fibers, the fiber diameter should be 1.0-30 µm, preferably 1.0-20 µm, and more preferably 1.5-10 µm.

Additionally, the above-mentioned materials forming the filter can be modified on the surface, and a type which does not inhibit the passage of non-nucleated erythrocytes and improves the retention of leukocytes is preferably used. The fetal nucleated cells and leukocytes retained in the filter can be recovered by cleansing the filter. For example, since immature nucleated erythroblasts have a greater tendency to detach than do the leukocyte components, the targeted fetal nucleated cells can be selectively recovered by passing a detergent fluid through the filter in the direction opposite that used when filtering the blood sample. As the detergent fluid used here, any biological buffer solution or biological saline solution such as a transfusion or a culture solution can be used as long as it is capable of detach the fetal nucleated cells.

Accordingly, the filter separating device in the separating system of the present invention may, in addition to the above-described filter and detergent fluid, comprise a fluid feeding device such as a syringe or pump for supplying a detergent fluid to the filter or a fluid reservoir for collecting non-nucleated erythrocyte components flushed form the filter. The sample containing fetal non-nucleated cells obtained in this way is made into the primary separated sample according to the present invention.

The primary separated sample which has been separated by filter can then be made to undergo the above-described panning process to further reduce the number of leukocytes, and this can also be used as the primary separated sample.

In the blood cell separating system of the present invention, a secondary separating device is offered for removing residual non-nucleated erythrocytes and leukocytes from primary separated materials obtained in the manner described above to obtain a secondary separated sample with concentrated fetal nucleated cells. In this secondary separating device, the primary separated .. samples are incubated on a substrate with glycoconjugate polymers affixed to the surface, together with a predetermined concentration of lectins under conditions in which the cells are inactivated, thereby performing a method for concentrating and attaching fetal nucleated cells contained in the primary separated samples onto the substrate by means of selective binding with the lectins due to lectin-carbohydrate interactions (hereafter referred to as the "carbohydrate-lectin method").

The details of the carbohydrate-lectin method are described in WO00/58443. As the glycoconjugate polymers used here, those which incorporate a carbohydrate chain structure on a hydrophobic polymer main chain such as polystyrene is used. For example, PVLA, PVMA, PVMan, PVMeA, PVLACOOH, PVG, PVGlcNac and PVLam as described in WO00/58443 are especially preferable. Of these, it is preferable to select those having a carbohydrate chain structure recognized by the lectins being used.

On a substrate which is surface-modified by these glycoconjugate polymers, the primary separated samples and lectins are incubated under conditions which inactivate the cells to form fetal nucleated cell-lectin complexes, which are concentrated and attached to the substrate by means of carbohydrate-lectin interaction.

The lectins used are preferably those which recognize the carbohydrate chains expressed by the fetal nucleated cells, examples including galactose-recognizing lectins such as SBA, PNA, ECL, AlloA and VAA, glucose-recognizing lectins such as Con A, LcH and PSA, and mannose-recognizing lectins such as LCA, GNA and CPA, but are not limited thereto. Here, by adjusting the amount of lectins added, the fetal nucleated cells can be made to attach at a higher rate, so as to allow selective precision separation in which intermixed leukocytes from the mother are not attached. Specifically, when using a plastic substrate, the amount of lectins added with respect to a primary separated sample containing about 2 × 10⁶ cells is preferably 8-35 µg, preferably 8-32 µg and more preferably 10-30 µg. Additionally, when using a glass substrate, the amount of lectins added with respect to a primary separated sample containing about 2 × 10⁶ cells is preferably 10-200 µg, preferably 20-100 µg and more preferably 30-75 µg. Thus, while the optimum range of lectin concentrations used will change somewhat depending on the material of the substrate and type of lectin employed, the optimum concentration could be selected by one skilled in the art without undue experimentation.

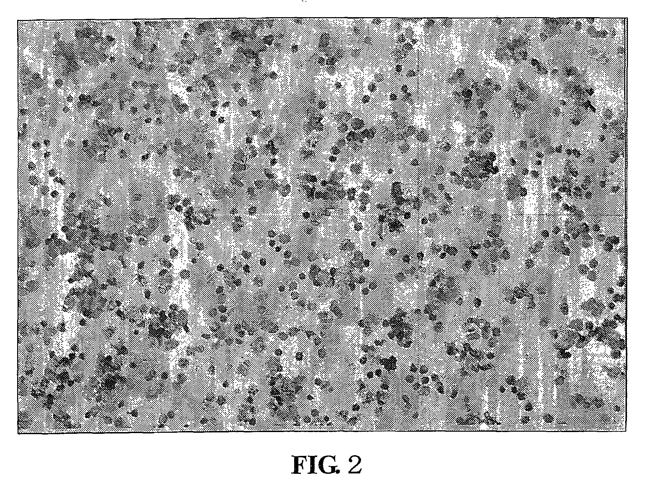

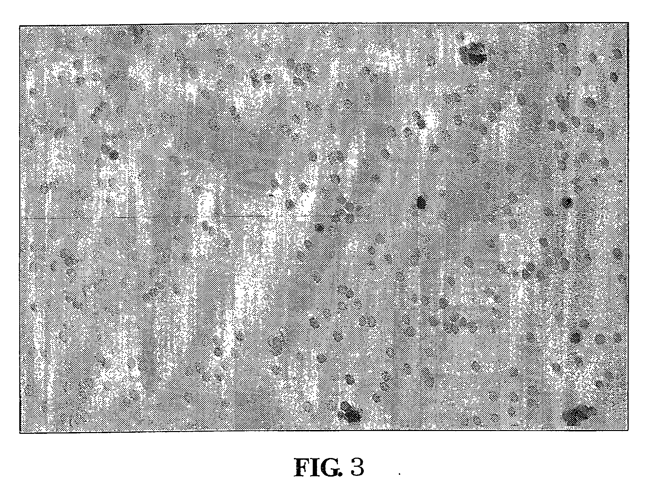

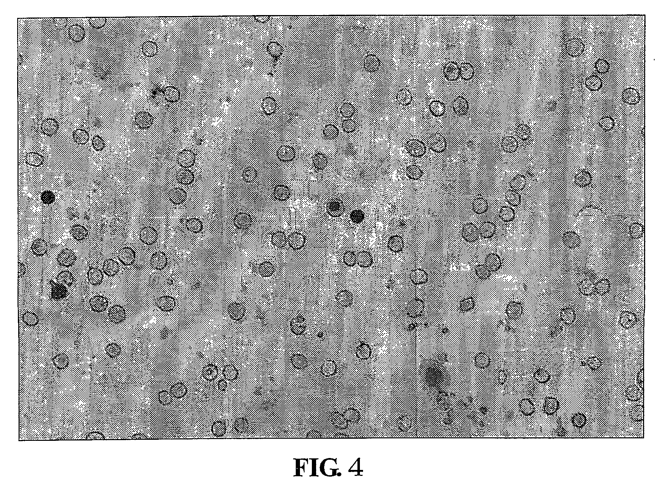

When immobilizing blood cells from a maternal blood sample onto a substrate coated with glycoconjugate polymer (PV-LA), in the case using 300 µg/ml of lectin; many leucocytes and the like (strongly stained cells) were contaminated as shown in the microscopic photograph of Figure 2, whilst in the case using 8 µg/ml of lectin, few unnecessary cells such as leucocytes existed and the erythrocytes were selectively adhered as shown in microscopic photographs of Figures 3 and 4.

The incubation of the lectins and cells is performed under conditions in which the cells are inactivated, and such conditions enable the above-described selectivity to be. "Conditions in which the cells are inactivated" refer to conditions in which the fluidity and self-adhesiveness of the cell membranes are lowered, and typically are such that the temperature is adjusted to at least 0 °C to 37 °C, preferably 0-36 °C, more preferably 4-30 °C and most preferably 4-22 °C. However, these conditions are not limited to the above-mentioned low-temperature adjustments, and for example can be achieved by adding a reagent which suspends cell respiration, such as sodium azide at 37 °C.

Additionally, the incubation time is not particularly specified, so long as it is sufficient for the cells and lectins to form cell-lectin complexes, but is typically 0-120 minutes, preferably 0-90 minutes, more preferably 0-60 minutes. Here, "0 minutes" indicates that the subsequent step is begun immediately after mixing the primary separated sample and the lectins.

In this secondary separation, the cells adhering to the substrate after incubation (fetal nucleated cells possibly including some non-nucleated erythrocytes) are separated by disposing of the unattached cells (leukocytes and the like) in the form of a cell suspension.

Accordingly, the secondary separating device according to the present invention comprises a substrate surface-modified with glycoconjugate polymers, lectins, and tools for inactivating the cells such as a cooling device or a reagent containing sodium azide, resulting in a secondary separated sample wherein fetal nucleated cells are attached to the substrate at a high density, and the unwanted components such as leukocytes are effectively removed.

In the blood cell separating system according to the present invention, the secondary separated sample, after being prepared by a preparing device to be explained, is finally made ready for genetic/chromosomal testing using the FISH method or the like. Therefore, by using the substrate in the second separating device as the slide plate in the FISH method or the like, a single substrate can be used for all processes from secondary separation to testing, which is very practical. Thus, if the slide plate used here is to be retained for microscopic examination, a flask, dish, cuvette or film having sufficient transparency so as not to inhibit the view in the microscope may be used, but when considering the general compatibility with microscopes, it is particularly preferable to use a chamber slide, to which a cover shell is attached during the secondary separation by lectins, the cover shell being removed after the secondary separation, and the attached fetal nucleated cells being dried and affixed, which can then be readily used directly in a FISH method.

The slide portion of the chamber slide may be made of any material which can be coated with the glycoconjugate polymers used in the above-mentioned carbohydrate-lectin process and viewed through a microscope, including organic materials such as polystyrene, polycarbonate, polysulfones, polyurethane and vinyl copolymers and the like, as well as inorganic materials such as glasses including silica. Additionally, when used for a FISH method, an organic solvent process at a high temperature must be used to denude the nuclei of the cells attached to the slide, so that a glass material which is not susceptible to deformation is preferably used.

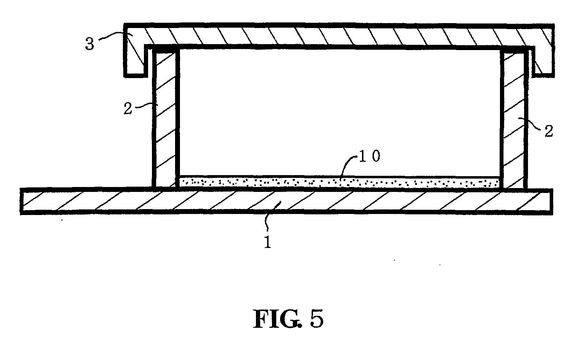

Figure 5 indicates an example of substrate usable in the secondary separation and subsequent processes in the blood cell separating system of the present invention. The substrate described in Figure 5 contains a slide portion (1) constituting the bottom of the chamber and side walls (2) placed perpendicular to the slide portion (1). A layer (10) of glycoconjugate polymer is formed on the upper surface of the bottom of the chamber. The opening of the chamber can be sealed by a cap (3).

The blood cell separating system of the present invention comprises a preparing device for centrifugating the secondary separated sample under predetermined conditions to make a preparation.

Generally, when preparing a cell preparation on a glass slide for chromosomal examination or the like, a cell suspension fluid is pipetted, dropped onto a slide and air-dried, or a sample containing cells is spread over the slide, which is then dried to form a smear sample (for example, see JP-A H7-27682). However, the present inventors discovered for the first time that in order to attach cells from the secondary separated sample to a slide in a form suitable for examination, special centrifugation conditions must be employed.

Thus, the preparing device of the present invention comprises a centrifuge apparatus for centrifugation of the secondary separated sample, which can centrifugate the secondary separated sample under predetermined conditions while still remaining on the substrate. The conditions differ according to the substrate (slide) which is used.

If the substrate is a plastic chamber slide, the cover shell is removed after the cell suspension is removed, but in order to prevent the slide surface from drying, it should preferably be centrifugated after cleansing with a biological buffer solution or FCS containing albumin abundant in proteins at close to the biological concentration. Furthermore, FCS diluted with distilled water by preferably 1/2, more preferably 3/5 can be used to spread attached cells widely over the plate, thus enabling them to be readily viewed through a microscope, and are therefore particularly preferable for use. The range of dilution may be of any range as long as it is such as not to decompose the cells.

As for the centrifugal force, 100-1500 G is preferable for attaching cells to the plate, and 400-700 G is more preferable for being effective in shortening the centrifugation time. While the centrifugation time will change according to the centrifugal force, it should generally be about 1-15 minutes. If directly air-dried without centrifugation, the cells can often atrophy so as to preclude the obtainment of a good cellular image.

On the other hand, if a glass chamber slide is used, it is preferable in the above-described centrifugation process to use a two-step procedure of running a first centrifugation with a low centrifugal force, and a second centrifugation at a higher centrifugal force. Additionally, it is preferable to replace the cell suspension containing the unattached cells with FCS of preferably 1/2, or more preferably 3/5 dilution and perform the first centrifugation prior to removing the cover shell. This first centrifugation is preferably a light centrifugation at 10-300 G, preferably 10-150 G, and more preferably 10-50 G, whereby it is possible to further prevent the attached cells from detaching or from deforming when pressed. Next, the cover shell is removed and the second centrifugation is performed, thereby obtaining an affixed cellular image appropriate for testing. The second centrifugation should be at a centrifugal force of 25-300 G, preferably 30-200 G, and more preferably 35-130 G, whereby the reproducibility of an affixed cellular image without deformation is further increased.

As described above, by performing a centrifugation process in accordance with specific conditions which differ according to the material of the slide substrate, it is possible to obtain a test preparation composed of a slide substrate on which fetal nucleated cells are affixed in a favorable state.

The blood cell separating system of the present invention further comprises an examination device for using test preparation in which fetal nucleated cells are concentrated and affixed to a substrate (slide) as described above for testing of the chromosomes and/or genes of the attached fetal nucleated cells.

The examination device may, for example, be a device for directly fluorescent labeling the chromosomes in the nuclei by means of the FISH method and viewing through a microscope, which is capable of detecting chromosome abnormalities such as aneuploidy, isochromosomes, translocation, deletion and reciprocal translocation according to the type of probe selected. Alternatively, the device may use the PCR method wherein the attached cells are recovered by peeling them by micromanipulation or laser dissection under a microscope, and amplifying the genes. The amplified genes can further be screened on a gene chip, and the system of the present invention can be adapted to various types of chromosomal/genetic diagnosis.

As described above, by using the blood cell separating system of the present invention, rare fetal nucleated cells in the maternal blood, which are difficult to separate and concentrate using specific gravity or surface marker identification by antibodies, are separated and concentrated at a high precision and high density by combining primary separation by density centrifugation or filter separation under specific conditions with secondary separation based on carbohydrate chain recognition by lectins under specific conditions. In particular, according to the present invention, these rare fetal nucleated cells are directly attached, separated and concentrated on a substrate which can then be used for testing of nucleic chromosomes and genes, with the cells attached to the substrate maintaining a good cell form adaptable to the genetic/chromosomal examination. Thus, the present invention offers a method of producing a test preparation of fetal nucleated cells capable of being directly used for testing in various chromosomal and/or genetic diagnoses by using the above-described system, as well as test preparations produced by the above-described method.

The method utilizing the system of the present invention is characterized by comprising a primary separation step of removing large amounts of unwanted cell components from rare cells contained in a sample, and a secondary separation step of separating and concentrating the rare cells with the object of further removing unwanted cell components from the primary separated samples, and for example, even if the secondary separation by the above-mentioned carbohydrate-lectin method is replaced by a separation method using antibodies specific to the rare cells, such a separation method and system would remain within the range of the present invention. Furthermore, aside from being fetal nucleated cells (fetal nucleated erythroblasts) intermixed in the maternal blood capable of diagnosing the state of and abnormalities in chromosomes and genes by testing after separation and concentration, the rare cells which are targeted may be leukemia cells remaining after remission, or immature cells in the umbilical blood, and may be any type of cell capable of being concentrated on the slide substrate by selection by lectins or antibodies as used in the crude cell separation system or precision separation system offered by the present invention. The term "fetal nucleated cell" as used in the present invention shall be interpreted to encompass all rare cells which are targeted in this way.

### Examples

Herebelow, the blood cell separation system of the present invention shall be described in detail with a focus on the setting of conditions for primary separation, secondary separation using the carbohydrate-lectin method, and preparing to maintain a good cell form.

### Example 1: Primary Separation by Density Centrifugation

Histopaque (Sigma) was obtained to use as a density centrifugation reagent, to which sodium diatrizoate was added and 6 types of density gradient fluid with specific gravities adjusted to 1.077-1.105 were prepared. 7 cc of venous blood were taken from women who were 10-20 weeks pregnant, then centrifugated for 30 minutes in each density gradient fluid (20 °C, 1500 rpm). The cells collected around the boundary between the density gradient fluid and plasma component (upper layer) were recovered, then centrifugally rinsed with a biological buffer solution to obtain a crude separated sample with most of the non-nucleated erythrocytes and platelets removed.

The samples primarily purified under the various density conditions were secondarily separated by the carbohydrate-lectin method. As the substrate, a plastic chamber slide (2 wells, product of Nalgenunc) was used. The glycoconjugate polymer coated onto the substrate was PVMeA, with 12 µg of lectins (SBA) added for about 2 × 10⁶ cells, then incubated for 30 minutes at 18 °C. The unattached cells were discarded in the form of a suspension, while the cells attached to the chamber slide were dried and stained with a Pappenheim stain. The stained cells were observed through a microscope, and the orthochromatic erythroblasts (fetal nucleated cells) which had been separated and attached to the slide were counted. The results are shown in Table 1.

**Table 1**

| | Number of erythroblasts detected after secondary separation using carbohydrate-lectin method on sample primarily separated by centrifugation with various fluids (average of 10 samples) | | | | | |
|---|---|---|---|---|---|---|
| | Density of Density Gradient Fluid | | | | | |
| | 1.077 | 1.080 | 1.090 | 1.095 | 1.100 | 1.105 |
| Number of orthochromatic erythroblasts detected on slide | 10.7 | 13.1 | 14.3 | 15.7 | 16.0 | 22.9 |
| Erythroblast detection ratio as compared with value for specific gravity 1.077 | 1.0 | 1.2 | 1.3 | 1.5 | 1.5 | 2.1 |

As shown in Table 1, increasing the density of the density gradient fluid (Histopaque) clearly increased the number of erythroblasts detected after carbohydrate-lectin precision separation. This made it dear that the conventional separation of blood cells by density centrifugation using a density gradient fluid having a specific gravity of 1.077 lost the erythrocytes with a high specific gravity. On the other hand, while the number of orthochromatic erythroblasts increases when the specific gravity exceeds 1.095, a considerable increase is also observed in the number of intermixed leukocytes, as a result of which there were cases in which the detection of erythroblasts by microscopy was inhibited by a reduction in the precision separation efficiency due to the carbohydrate-lectin method.

### Example 2: Additional Separation by Panning

A plastic chamber slide (4 wells, product of Nalgenunc) was treated with FCS or a 0.01 wt% aqueous solution of a glycoconjugate polymer (PV-Sugar) (product of Netech). As the glycoconjugate polymer, those having the structures of glucose, maltose, gluconic acid, N-acetylglucosamin, mannose, lactose or melibiose were used.

Density gradient centrifugation was performed on umbilical blood recovered after birth according to a standard method using Histopaque (d, 1.095), and the cells aggregating near the boundary between the Histopaque and plasma were collected. The samples were resuspended in RPMI1640 to which 10 wt% FCS was added, and inoculated onto the above-described wells whose surfaces were coated with FCS or glycoconjugate polymers. After incubation for 30 minutes at 37 °C, the unattached cells were recovered in the form of a cell suspension fluid, and the cells attached to the wells were stained with a Pappenheim stain to identify their types. The results are shown in Table 2. Table 2 shows the ratio between the erythrocyte fraction (including erythroblasts) and leukocyte fraction attached to the well surfaces with the respective types of coating, as well as the rate of adhesion of all inoculated cells.

**Table 2**

| | Adhesion of Blood Cells to Respective Wells (average of 5 samples) (%) | | | | | | | |
|---|---|---|---|---|---|---|---|---|
| | FCS | Mannose | Glucose | Maltose | Gluclonic Acid | Glucosamin | Lactose | Melibiose |
| Leukocyte / Erythrocyte Ratio | 99.2 | 99.1 | 98.9 | 99.5 | 99.1 | 97.4 | 98.9 | 98.9 |
| Overall Adhesion Rate | 69.3 | 71.0 | 62.3 | 64.6 | 78.0 | 72.6 | 65.8 | 78.1 |

As shown in Table 2, it is clear that when panning with wells having their plastic surfaces coated with blood serum proteins (FCS) or glycoconjugate polymers, the cells of almost all blood corpuscles which attach are leukocytes. The attachment of the erythrocyte fraction is inhibited in wells treated with maltose, gluconic acid, FCS and mannose, while gluconic acid, glucosamin and mannose excel in the overall rate of adhesion which indicates the removability of leukocytes. In this case, no erythroblasts were included in the attached erythrocyte fraction with the exception of a portion of he glucose type materials in which about 1 or 2 attached erythroblasts were observed.

Next, FCS was selected as a treatment agent for removing a suitable amount of leukocytes without much loss of the erythrocyte fraction, and the treatment effects of maternal blood were studied. A primarily separated sample obtained by density centrifugation using a density gradient fluid with a specific gravity of 1.095 was divided into two portions, one of which was panned under the above-described conditions and the other of which was not treated, then made to undergo a carbohydrate-lectin secondary separation under the same conditions as Example 1.

**Table 3**

| | Effects of panning on lectin secondary separation (average of 20 samples) (ratio compared to case of no panning) |
|---|---|
| Erythrocyte/Leukocyte Ratio per Field of Slide | 2.6 times |
| Number of Erythrocytes Detected on Slide | 2.6 times |

The erythrocyte/leukocyte ratio per field of the slide in Table 3 indicates the rate of removal of leukocytes in secondary separation, and the number of erythrocytes detected on the slide indicates the erythroblast recovery efficiency. Specifically, the results of a count of attached cells under the microscope are shown as a comparative ratio (multiple) of the cases where there is no panning and the case where panning has been performed. From the results in Table 3, it is dear that panning improves the selective separation and concentration efficiency of erythroblasts in the carbohydrate-lectin method. This is believed to be caused by a synergistic effect due to the fact that the panning has removed excess leukocytes, thus enabling the cells (erythroblasts) to be attached by the lectins to efficiently interact with the lectins so as to reduce adhesion misses, and that miscounting of erythroblasts has been reduced due to a decrease in the number of nucleated cells other than the erythroblasts during microscope observation.

The results from Table 3 show that even when compared with the case of a specific gravity of 1.095 in Table 1, the intermixture of leukocytes which block microscope observation is inhibited by panning, while making it possible to detect more erythroblasts than in the case of a specific gravity of 1.105 (no panning). Additionally, in the maternal blood, there was almost no loss of erythroblasts due to panning.

### Example 3: Primary Separation by Filter Separation

Instead of the density centrifugation method of Example 1, a primary separation was performed using a filter comprising an unwoven polyester fabric with an average pore size of 8 µm. A sample of maternal blood was diluted with a biological buffer solution containing 1 wt% BSA, then passed through the filter by natural dripping. Next, the buffer solution alone was passed through a filter to rinse away the residual erythrocytes in the filter. Subsequently, the buffer solution was passed in the opposite direction with a syringe pump, and the unattached cells which did not pass through the filter were recovered. The cell fraction which did not pass through the filter but did not strongly adhere to the filter was taken as the primary separated sample, which was secondarily separated by the carbohydrate-lectin method. Table 4 shows the results with a primary separated sample obtained from the above-described filter, in the form of a comparative ratio (multiple) with respect to the results for the case where the cell fraction recovered by FCS panning is secondarily separated by the carbohydrate-lectin method.

**Table 4**

| | Effects of filtering on lectin secondary separation (average of 20 samples) (ratio compared to case of density centrifugation + panning) |
|---|---|
| Erythrocyte/Leukocyte Ratio per Field of Slide | 1.5 times |
| Number of Erythrocytes Detected on Slide | 2.4 times |

According to Table 4, both the erythrocyte/leukocyte ratio per field of the slide (leukocyte removal rate in secondary separation) and the number of erythroblasts detected on the slide (erythroblast recovery rate in secondary separation) are improved, thus dearly indicating that the selective separation and concentration efficiency of erythroblasts with the carbohydrate-lectin method can be improved by the filter process. The primary separation by the filter is believed to be due to non-nucleated erythrocytes which are capable of deforming being passed through the filter, and cells which are neither passed nor trapped by the filter being recovered by rinsing the filter. Thus, it was demonstrated that the loss of erythroblasts is further suppressed by using a primary separating method rather than the density centrifugation method as the filtering method. While the erythrocyte/leukocyte selectivity was held to just a slight improvement, this is due to the fact that non-nucleated erythrocytes remaining in the filter were recovered by rinsing.

### Example 4: Centrifugation in Preparing Step

A glass chamber slide (product of Nalgenunc) which is suitable for the FISH method was used to perform a secondary separation with the carbohydrate-lectin method. As for the primary separating conditions, the following were employed (same as Example 2).
Density Centrifugation: d, 1.095
FCS Panning

Comparisons were made between cases in which secondary separation by the carbohydrate-lectin method was followed by cases in which the cell suspension containing unattached cells was discarded, the chamber replaced with FCS, the cover shell removed, and the slide centrifugated (Experimental Conditions 1 and 2), and cases in which the cell suspension containing unattached cells was discarded, the chamber replaced with FCS, a first centrifugation immediately performed, and a second centrifugation performed on the slide after removing the cover shell (Experimental Conditions 3-11).

As the FCS, the raw liquid (1/1) was diluted by 1/2 with distilled water before use, and after the second centrifugation, the slides were air-dried at standard temperature. The cells on the slide were stained with a Pappenheim stain, and their respective stain appearances were compared. The results are shown in Table 5.

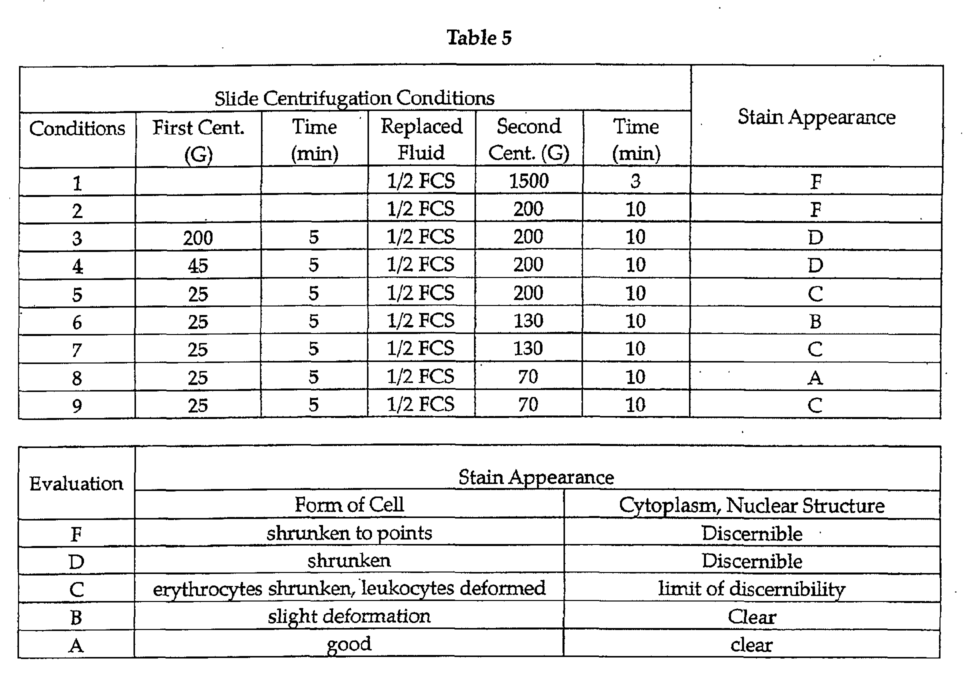

Unlike normal smear samples, the cells on a slide which have been secondarily separated by the carbohydrate-lectin method need to have the cells attached by centrifugation pressed, but when a glass slide is used as the substrate, a first centrifugation must be made in FCS solution at a low speed. Even in the second centrifugation after removal of the cover shell, relatively low-speed conditions retained good stain appearances. Additionally, while FCS- or BSA-added buffer solutions with a high protein concentration are effective as replacement fluids, diluted FCS under biological conditions induced swelling of the attached cells, and retained better stain appearances: For example, cells indicated suitable morphologies and dear structures of cytoplasm and nuclei under conditions 8, 10 and 11. In particular, under condition 11, in which 3/5 diluted FCS was used, almost no variation effects from samples such as storage periods or individuals were observed. When using plastic slides, an adequately good stain appearance was obtained under the above-given Condition 1.

### Effects of the Invention

According to the blood separating system of the present invention, the nucleated erythrocytes which are fetal nucleated cells that are contained in the maternal blood in extremely small amounts are selectively separated, concentrated and attached to a substrate. Additionally, by appropriately selecting the substrate used, the processing from the secondary separation step to the preparing step can be performed on a single substrate to obtain a test preparation which is suitable for chromosomal/genetic diagnosis, and this test preparation can be directly applied to testing means such as the FISH method. Accordingly, a fetal nucleated cell test sample with a high clinical value for prenatal diagnosis can be produced conveniently and at a low cost without invading the maternal body.

## Claims

1. A blood cell separating system comprising:
a primary separating device for removing mainly non-nucleated erythrocytes, leukocytes and platelets from a blood sample taken from a pregnant woman to obtain a primary separated sample;
a secondary separating device for removing residual non-nucleated erythrocytes and leukocytes from the primary separated sample obtained from said primary separating device, to obtain thereby a secondary separated sample with concentrated fetal nucleated cells, the secondary separating device involving incubation of said primary separated sample, under conditions which inactivate the cells, along with a predetermined concentration of lectins, on a substrate having glycoconjugate polymers affixed to the surface thereof, thereby selectively binding fetal nucleated cells contained in said primary separated sample with said lectins to thereby concentrate and attach them to said substrate by means of a lectin-carbohydrate interaction to form a secondary separated sample; and
preparing device for preparing the secondary separated sample obtained from said secondary separating device, the preparing device involving centrifugation, under predetermined conditions, of said substrate on which said fetal nucleated cells have been concentrated and attached, to thereby obtain a test preparation.

2. A blood cell separating system in accordance with claim 1, wherein said primary separating device is a density centrifugation device using a density gradient fluid having a density at least exceeding 1.077 mg/cm³.

3. A blood cell separating system in accordance with claim 2, further comprising panning device for removing leukocytes intermixed in the sample containing fetal nucleated cells obtained by said density centrifugation device.

4. A blood cell separating system in accordance with claim 1, wherein said primary separating device is a device for separating non-nucleated erythrocytes as well as leukocytes using a filter.

5. A blood cell separating system in accordance with claim 1, wherein the conditions which inactivate the cells in said secondary separating device are low-temperature conditions of at least 0 °C and less than 37 °C, or conditions which suspend cell respiration.

6. A blood cell separating system in accordance with claim 1, wherein the centrifugation conditions in said preparing device are centrifugation for 1-15 minutes at 100-1500 G when using a plastic substrate, and a first centrifugation of 1-10 minutes at 10-300 G-followed by a second centrifugation of 5-15 minutes at 25-300 G when using a glass substrate.

7. A blood cell separation system in accordance with claim 1, wherein a substrate used in said preparing device is obtained by substituting the suspension contained in the secondary separated sample with FCS diluted to 1/2, preferably 3/5.

8. A blood cell separating system in accordance with claim 1, further comprising an examination device for testing the chromosomes and/or genes in fetal nucleated cells contained in a test preparation obtained from said preparing device.

9. A blood cell separating system in accordance with claim 8, wherein said examination device comprises a staining tool for staining chromosomes in the nuclei of fetal nucleated cells contained in the preparation.

10. A blood cell separating system in accordance with claim 9, wherein the staining of the nuclei in said staining means is due to the FISH method.

11. A blood cell separating system in accordance with claim 8, wherein said examination device includes a chromosome testing device using an optical microscopy or a fluorescent microscopy.

12. A blood cell separating system in accordance with claim 8, wherein said examination device comprises tools for amplifying genes extracted from the chromosomes of fetal nucleated cells contained in the preparation.

13. A blood cell separating system in accordance with claim 12, wherein said amplifying tools involve materials for performing the PCR method.

14. A blood cell separating system in accordance with claim 12, wherein said examination device contains a device for screening amplified genes.

15. A method for producing a test preparation for prenatal fetal chromosomal and/or genetic diagnosis, comprising:
a primary separating step of removing mainly non-nucleated erythrocytes, leukocytes and platelets from a blood sample taken from a pregnant woman to obtain a primary separated sample;
a secondary separating step of incubating said crude separated sample, under conditions which inactivate the cells, along with a predetermined concentration of lectins, on a substrate having glycoconjugate polymers affixed to the surface thereof, thereby selectively binding fetal nucleated cells contained in said primary separated sample with said lectins to thereby concentrate and attach them to said substrate by means of a lectin-carbohydrate interaction, so as to selectively remove residual leukocytes in said primary separated sample to obtain a secondary separated sample having the. desired fetal nucleated cells in concentrated form; and
a preparing step of performing centrifugation, under predetermined conditions, of the secondary separated sample in which said fetal nucleated cells have been concentrated and attached.

16. A method in accordance with claim 15, wherein said primary separating step is performed by density centrifugation using a density gradient fluid having a density at least exceeding 1.077 mg/cm³.

17. A method in accordance with claim 16, further comprising a step of panning the sample containing fetal nucleated cells obtained by said density centrifugation means to remove intermixed leukocytes.

18. A method in accordance with claim 15, wherein said primary separating step is performed by separating non-nucleated erythrocytes as well as leukocytes using a filter.

19. A method in accordance with claim 15, wherein the conditions which inactivate the cells in said precision separating step are low-temperature conditions of at least 0 °C and less than 37 °C, or conditions which suspend cell respiration.

20. A method in accordance with claim 15, wherein the centrifugation conditions in said preparing step are centrifugation for 1-15 minutes at 100-1500 G when using a plastic substrate, and a first centrifugation of 1-10 minutes at 10-300 G followed by a second centrifugation of 5-15 minutes at 25-300 G when using a glass substrate.

21. A method in accordance with claim 15, wherein a substrate in which the suspension contained in the secondary separated sample in substituted with FCS diluted to 1/2, preferably 3/5 is provided to said preparing step.

22. A test preparation for prenatal fetal chromosomal and/or genetic diagnosis produced by a method in accordance with any one of claims 15-21.

23. A substrate for a blood cell separating system in accordance with any one of claims 1-14, having glycoconjugate polymers affixed to the surface thereof.

24. A blood cell separating method comprising a primary separating step of removing most of the unneeded cellular components included in a sample containing rare cells; and a secondary separating step of separating and concentrating the targeted rare cells by further removing unneeded cell components from the primary separated sample.
